# EUROPEAN PATENT SPECIFICATION

(11) **EP 1 635 792 B1**
(45) Date of publication and mention of the grant of the patent: **04.03.2009**
(21) Application number: 04756130.3
(22) Date of filing: 24.06.2004
(51) Int. Cl.: A61K 9/26, A61K 9/16, A61K 31/403

(54) **STABLE PHARMACEUTICAL COMPOSITIONS OF 2-AZA-BICYCLO¬3.3.0 -OCTANE-3-CARBOXYLIC ACID DERIVATIVES**
STABILE PHARMAZEUTISCHE ZUBEREITUNGEN MIT 2-AZA-BICYCLO¬3.3.0 -OCTAN-3-CARBOXYLSÄURE-DERIVATEN
COMPOSITION PHARMACEUTIQUE STABLE DE DERIVES DE L'ACIDE 2-AZA-BICYCLO¬3.3.0 -OCTANE-3-CARBOXYLIQUE

(30) Priority: 26.06.2003 US 482518 P
(43) Date of publication of application: 22.03.2006
(73) Proprietor: Teva Pharmaceutical Industries Limited, Petah Tikvah 49131 (IL)
(72) Inventor: HRAKOVSKY, Julia, 48057 Rosh-Ha-Ayin (IL); TENENGAUZER, Ruth, 45322 Hod Hasharon (IL)
(74) Representative: Gallagher, Kirk James
(86) International application number: PCT/US2004/020484
(87) International publication number: WO 2005/002548

(56) References cited:
- EP-A- 0 280 999
- US-A- 4 830 853
- US-A- 5 006 344
- US-A- 6 086 919
- US-A1- 2002 131 999
- US-A1- 2003 068 366
- US-B1- 6 417 196

## Description

### FIELD OF THE INVENTION

This invention relates to stabilized compositions of 2-aza-bicyclo[3.3.0]-octane-3-carboxylic acid derivatives and methods for preparing them.

### BACKGROUND OF THE INVENTION

Ramipril, quinapril, moexipril, fosinopril, enalapril, perindopril, and trandolapril are examples of 2-aza-bicyclo[3.3.0]-octane-3-carboxylic acid derivatives used in pharmaceutical formulations. Ramipril, which has the chemical name (2S, 3aS, 6aS)-1[(S)-N-[(S)-1-carboxy-3-phenyl-propyl] alanyl]octa hydrocyclopenta [b]pyrrole-2-carboxylic acid, 1-ethyl ester, is a pro-drug oframiprilat, the active form of this angiotensin-converting enzyme (ACE) inhibitor.

Ramipril and certain other ACE inhibitors are reported to be effective antihypertensive drugs, but they are often susceptible to degradation. Ramipril is believed to degrade into two main products: diketopiperazine (DKP) and ramiprilat. Decomposition during manufacture and storage may adversely affect the effectiveness of the drug product or may cause the drug product to deviate from regulatory purity or potency requirements. It is therefore desirable to increase the stability of 2-aza-bicyclo[3.3.0]-octane-3-carboxylic acid derivative formulations.

The following chemical structures are some examples of 2-aza-bicyclo[3.3.0]-octane-3-carboxylic acid derivatives and their corresponding active form degradants.

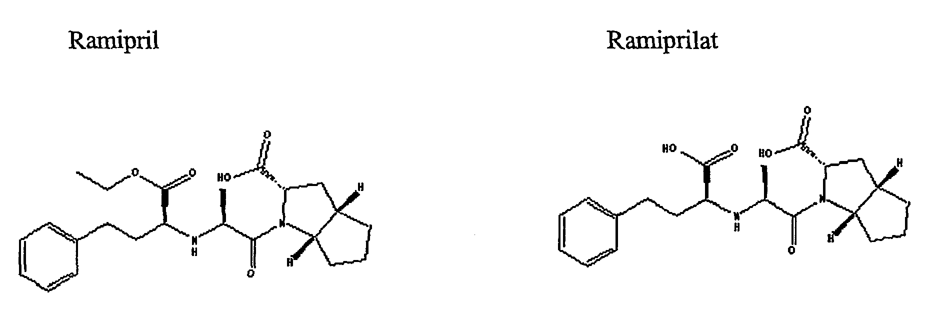

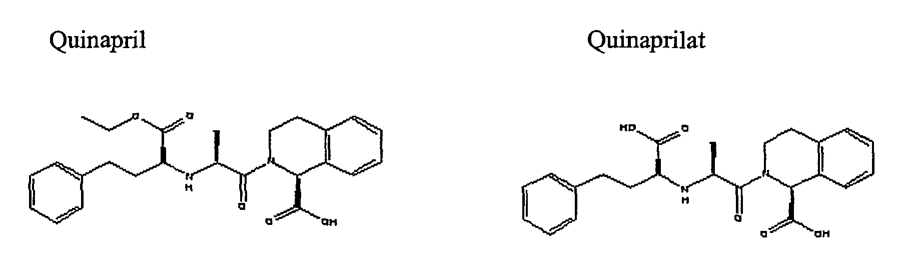

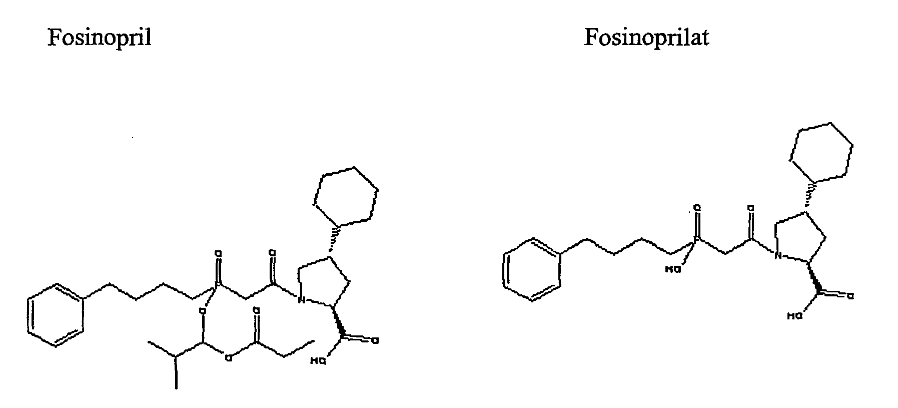

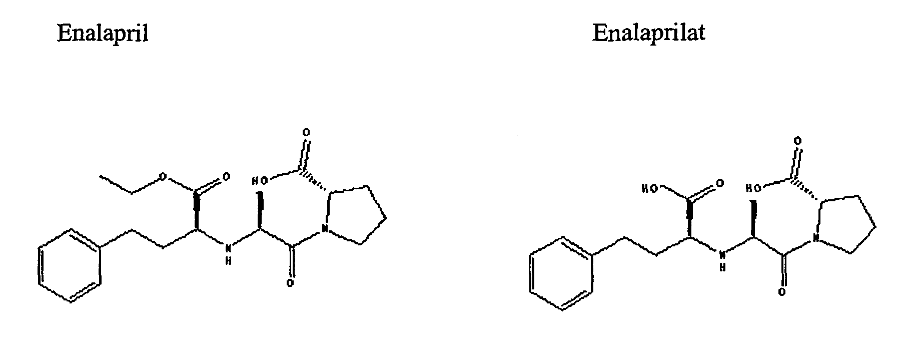

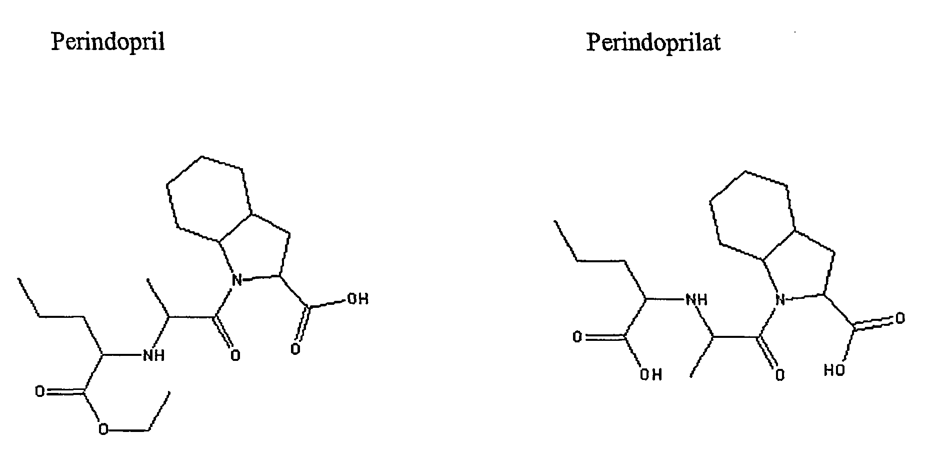

EP 280,999 B1 (Jan. 7, 1993) describes stabilized pharmaceutical compositions that contain ACE inhibitors, an alkali or alkaline earth metal carbonate, and a saccharide.

EP 317,878 B1 (April 8, 1992) is directed towards stabilized compressed pharmaceutical formulations that may contain ramipril.

U.S. Patent No. 6,417,196 is directed to ACE inhibitor-containing compositions stabilized by the presence of magnesium oxide.

U.S. Patent No. 4,834,853 is directed towards the oxidation- and color-stability of certain ACE inhibitors.

U.S. Patent No. 4,793,998 is directed towards minimizing cyclization and hydrolysis of certain ACE inhibitors.

US Patent No. 5,006,344 describes the improved stability of fosinopril in a tablet formulation by exchanging the lubricant magnesium stearate with sodium stearyl fumarate.

### SUMMARY OF THE INVENTION

In one embodiment, the present invention provides a stable pharmaceutical composition comprising an intimate admixture including a 2-aza-bicyclo[3.3.0]-octane-3-carboxylic acid derivative and an effective amount of a lubricant to stabilize the composition, and at least one external excipient. In a preferred embodiment, the external excipient is in powder form.

In another embodiment, the present invention provides a method for preparing a stable pharmaceutical composition comprising forming an intimate admixture including an 2-aza-bicyclo[3.3.0]-octane-3-carboxylic acid derivative and an effective amount of a lubricant. The method further comprises blending the intimate admixture with an external excipient.

### DETAILED DESCRIPTION OF THE INVENTION

### Definitions

As used herein, the term "stable pharmaceutical composition" refers to a pharmaceutical composition according to the invention that is less susceptible to degradation than a similar composition not having an intimate admixture of a derivative and a stabilizing effective amount of lubricant.

The term "effective amount" refers to possible weight percentages that will produce the intended effect of stabilizing the composition.

The term "by weight," unless otherwise specified, means by weight of the total composition.

The term "by weight of the derivative" means by weight of the 2-aza-bicyclo[3.3.0]-octane-3-carboxylic acid derivative before degradation of the derivative.

The term "intimate admixture" refers to a mixture of closely-packed components, such as those exemplified herein, as opposed to a simple blend. An intimate admixture can be obtained, for example, by co-precipitation, co-milling, compression, granulation, or the like.

The term "external excipient" refers to an excipient or combination of excipients that have not been intimately admixed with a derivative.

The term "derivative" refers to a 2-aza-bicyclo[3.3.0]-octane-3-carboxylic acid derivative. 2-aza-bicyclo[3.3.0]-octane-3-carboxylic acid derivatives include, for example, ramipril, quinapril, moexipril, fosinopril, enalapril, perindopril, and trandolapril.

The term "principal degradant" refers to the single degradant from a 2-aza-bicyclo[3.3.0]-octane-3-carboxylic acid derivative with the highest percentage by weight. For example, the principal degradant of ramipril is usually diketopiperazine.

The term "active form degradant" refers to the active compound that 2-aza-bicyclo[3.3.0]-octane-3-carboxylic acid derivatives degrade into. For example, the active form degradant of ramipril is ramiprilat.

The term "DKP" refers to diketopiperazine.

In one embodiment, the present invention provides a stable pharmaceutical composition comprising (a) an intimate admixture including a 2-aza-bicyclo[3.3.0]-octane-3-carboxylic acid derivative and an effective amount of a lubricant to stabilize the composition, and (b) at least one excipient.

With respect to the intimate admixture of the stable pharmaceutical composition of the present invention, the derivative of the intimate admixture is preferably selected from the group consisting of ramipril, quinapril, moexipril, fosinopril, enalapril, perindopril, and trandolapril. The amount of the derivative is preferably from about 0.3% to about 6% by weight. More preferably, the derivative is present in the amount of from about 0.8% to about 5% by weight, and most preferably from about 0.8% to about 4.2% by weight.

The lubricant of the intimate admixture can be selected from the group consisting of magnesium stearate, talc, stearic acid, glycerylbehenate, polyethylene glycol, ethylene oxide polymers, sodium lauryl sulfate, magnesium lauryl sulfate, sodium oleate, sodium stearyl fumarate, DL-leucine, and others known in the art. Preferably, the lubricant is sodium stearyl fumarate. The effective amount of lubricant in the intimate admixture is preferably from about 0.3% to about 60% by weight, more preferably from about 0.8% to about 50% by weight, more preferably from about 1% to about 40% by weight, and more preferably from about 2% to about 10% by weight of the intimate admixture.

The intimate admixture can also include processing agents or other excipients that do not significantly adversely affect the stability of the composition. Thus, it is desirable to minimize the number and quantity of these additional excipients in the intimate admixture. In one embodiment, the intimate admixture further comprises one non-lubricant excipient. Preferably, the non-lubricant excipient is in the amount of about 95% by weight of the intimate admixture, or less, preferably less than about 50%.

Examples of possible excipients are spray-dried monohydrate lactose or anhydrous lactose, sucrose, dextrose, mannitol, sorbitol, starch, pregelatinized starch (e.g. starch 1500), cellulose (e.g. microcrystalline cellulose; Avicel), dihydrated or anhydrous dibasic calcium phosphate (available commercially under the registered trademark Emcompress from Mendell or A-Tab and Di-Tab from Rhone-Poulenc, Inc., Monmouth Junction, N.J.), calcium carbonate, calcium sulfate, and others as known in the art. To improve flowability, a preferred excipient is microcrystalline cellulose, preferably in the amount of about 30% or less by weight of the total composition.

The intimate admixture can also include disintegrants, binders, coloring agents, buffering agents, and other commonly employed pharmaceutically acceptable agents, provided they do not cause substantial degradation of the derivative, which is believed to be particularly sensitive to acidic agents.

With respect to the external excipient of the stable pharmaceutical composition of the present invention, the external excipient may include one or more excipients, such as processing agents. A preferred processing agent is microcrystalline cellulose. Preferably, the external excipient is in the amount of from about 20% to about 99% by weight, more preferably from about 40% to about 98% by weight, and more preferably from about 50% to about 90% by weight.

The external excipient may include, for example, a lubricant, such as those described herein. A preferred lubricant is sodium stearyl fumarate or magnesium hydroxide. Preferably, the amount of lubricant in the external excipient, if any, is in the amount of from about 0.3% to about 10% by weight, more preferably from about 0.5% to about 3% by weight, and more preferably from about 0.8% to about 2% by weight.

Alternatively or additionally, the external excipient may also include disintegrants, binders, coloring agents, buffering agents, and/or other commonly employed pharmaceutically acceptable agents.

Examples of suitable disintegrants are starch, pregelatinized starch, sodium starch glycolate, sodium carboxymethylcellulose, crosslinked sodium carboxymethylcellulose (e.g., sodium croscarmellose; crosslinked starch available under the registered trademark Ac-Di-Sol from FMC Corp., Philadelphia, Pa.), clays (e.g., magnesium aluminum silicate), microcrystalline cellulose (such as those available under the registered trademark Avicel from FMC Corp. or the registered trademark Emcocel from Mendell Corp., Carmel, N.Y.), alginates, gums, surfactants, effervescent mixtures, hydrous aluminum silicate, cross-linked polyvinylpyrrolidone (available commercially under the registered trademark PVP-XL from International Specialty Products, Inc.), and others as known in the art.

Examples of suitable binders include, e.g., acacia, cellulose derivatives (such as methylcellulose and carboxymethylcellulose, hydroxypropylmethylcellulose, hydroxypropylcellulose, hydroxyethylcellulose), gelatin, glucose, dextrose, xylitol, polymethacrylates, polyvinylpyrrolidone, starch paste, sucrose, sorbitol, pregelatinized starch, gum tragacanth, alginic acids and salts thereof such as sodium alginate, magnesium aluminum silicate, polyethylene glycol, guar gum, bentonites, and the like.

Coloring agents may include titanium dioxide and/or dyes suitable for food such as those known as FD & C dyes and natural coloring agents such as grape skin extract, beet red powder, beta carotene, annato, carmine, turmeric, paprika, and so forth.

Examples of possible buffering agents include tribasic sodium phosphate, anhydrous sodium carbonate, glycine, magnesium hydroxide, and the like.

In another embodiment, the stable pharmaceutical composition further includes a diuretic agent. Acceptable diuretic agents include high-ceiling diuretics, furosemide, bumetanide, ethacrynic acid, torsemide, muzolimide, azosemide, piretanide, tripamide, chlorothiazide, hydrochlorothiazide, chlorthalidone, indapamide, metozalone, cyclopenthiazide, xipamide, mefruside, dorzolamide, acetazolamide, methazolamide, ethoxzolamide, cyclothiazide, clopamide, dichlorphenamide, hydroflumethiazide, trichlormethiazide, polythiazide and benzothiazide. A preferred diuretic agent is hydrochlorothiazide. The preferred amount of the diuretic agent, when present, is from about 0.5% to about 40% by weight, more preferably from about 1% to about 30% by weight, and more preferably from about 2% to about 15% by weight.

Preferably, the stable pharmaceutical composition of the present invention resists degradation when stored under high stress conditions. For example, when stored at 55°C for 48 hours, preferably (1) the total amount of the principal degradant and the active form degradant is about 3.3% by weight of the derivative, or less, more preferably about 1% by weight of the derivative, or less; (2) the amount of the principal degradant present is about 3% by weight of the derivative, or less, more preferably about 1% by weight of the derivative, or less; and/or (3) the amount of the active form degradant present is about 0.3% by weight of the derivative, or less, more preferably about 0.2% by weight of the derivative, or less.

For example, for the derivative ramipril, when stored at 55°C for 48 hours, (1) the total amount of DKP and ramiprilat is preferably about 3.3% by weight of the ramipril, or less, more preferably about 1% by weight of the ramipril, or less; (2) the amount of DKP present is preferably about 3% by weight of the ramipril, or less, more preferably about 1% by weight of the ramipril, or less; and/or (3) the amount of ramiprilat present is preferably about 0.3% by weight of the ramipril, or less, more preferably about 0.2% by weight of the ramipril, or less.

A stable pharmaceutical composition of the derivatives can be prepared by, first, forming an intimate admixture comprising a derivative and a lubricant. Second, the intimate admixture can be blended with an external excipient to form a final blend that is preferably transformed into solid unit dosage form, such as a tablet or capsule.

Preferably, the intimate admixture is in granular form. Granules can be formed, for example, by dry granulation or wet granulation. Wet granulation techniques are known in the art and involve mixing the ingredients with a solvent, such as ethanol or isopropyl alcohol, and drying the mixture to obtain granules. Dry granulation can be performed, for example, by compaction or slugging. Compaction techniques are well known in the art and typically include the use of a roller compactor. Slugging is a common technique in the field and involves the use of a tableting machine to produce slugs and passing the slugs through a mill or an oscillating granulator to form granules. Typical screen aperture sizes are, for example, 0.5 mm, 0.8 mm, or 1.0 mm.

The stable pharmaceutical composition of the present invention is preferably in solid unit dosage form, more preferably in tablet or capsule form. Conventional tableting processes can be employed, e.g., by forming a tablet from a desired mixture of ingredients into the appropriate shape using a conventional tablet press. Tablet formulation and processing techniques are generally known in the field. Capsule formulation methods are also commonly known in the art.

The functions and advantages of these and other embodiments of the present invention will be more fully understood from the examples below. The following examples are intended to illustrate the benefits of the present invention, but do not exemplify the full scope of the invention.

### EXAMPLES

### Examples 1-2: Dry Granulation

The following tablets were prepared by dry granulation. The ingredients of Part I of Table 1 were blended, and initial compression was carried out using a rotary tableting machine. The compressed material was milled through an oscillating granulator to produce granules having an average diameter of about 0.8 mm. The external excipient was added (Part II) and the final blend was then compressed to form tablets.

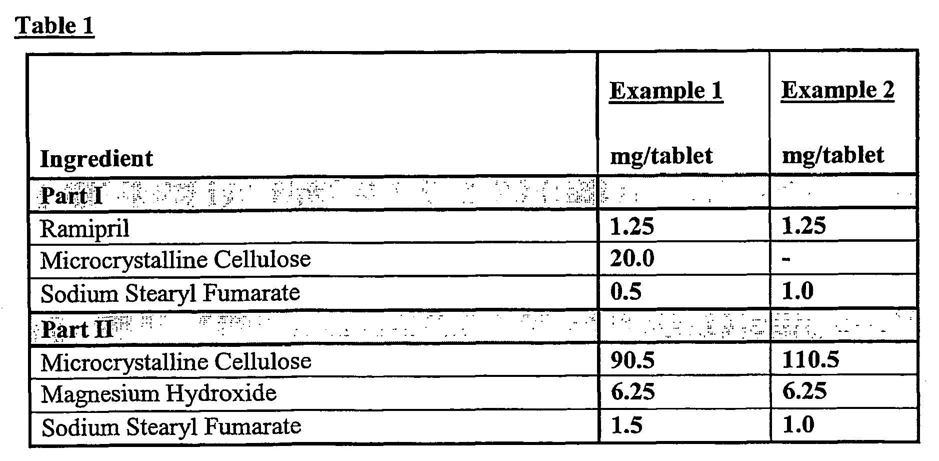

### Comparative Examples 3-5: Direct Compression

The ingredients in Table 2 were blended and compressed into tablets.

**Table 2**

| | **Example 3** | **Example 4** | **Example 5** |
|---|---|---|---|
| **Ingredient** | **mg/tablet** | **mg/tablet** | **mg/tablet** |
| Ramipril | **1.25** | **1.25** | **1.25** |
| Microcrystalline Cellulose | **115.3** | **112.75** | **100.75** |
| Methocel E-5^{™} | **-** | **4.0** | **-** |
| Povidone (PVP K-30) | **-** | **-** | **3.0** |
| Magnesium Hydroxide | **6.25** | **-** | **-** |
| Colorant | **0.2** | **-** | **-** |
| Crospovidone | **-** | **-** | **13.0** |
| Sodium Stearyl Fumarate | **2.0** | **2.0** | **2.0** |

### Comparative Examples 6-7: Wet Granulation

The ingredients of Part I of Table 3 were granulated using isopropyl alcohol as a granulation liquid. The granulate was dried, milled and blended with ingredients from Part II. The final blend was compressed into tablets.

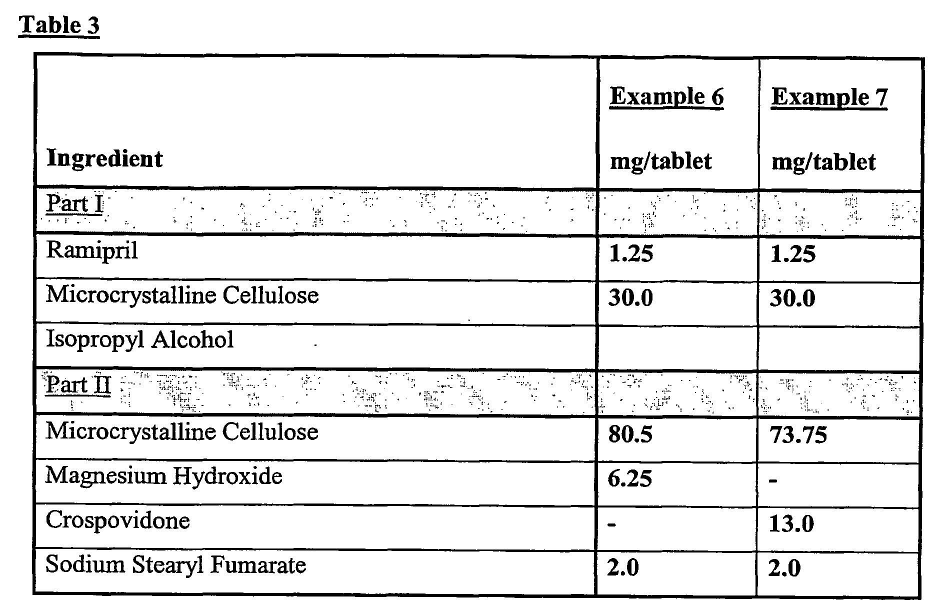

### Results

A stability test was performed on each sample by packing the tablets in plastic containers and storing them in the oven at 55°C with added water. After 48-hour storage, the amount of ramiprilat and DKP present were measured. High performance liquid chromatography (HPLC) was employed with the following parameters:
Column: Zorbax SB C-8, 5µm, 250 × 4.6 mm
Mobile Phase: Buffer adjusted to pH 2.00 with acetonitrile (65:35 V/V)
Flow Rate: 1.0 mL / min
Detection: UV, λ = 215 nm
Column Temp.: 60°C
Sample Temp.: 4°C
Injection Volume: 50 µl

A stability test was also conducted for the marketed product Tritace® 1.25 mg, which is reported to contain ramipril, starch, microcrystalline cellulose, sodium stearyl fumarate, hypromellose, and colorant.

The results are shown in Table 4.

**Table 4**

| **Example** | **Ramiprilat (%)** | **DKP (%) after** | **Total % of DKP and** |
|---|---|---|---|
| **No.** | **after 48h at 55°C** | **48h at 55°C** | **Ramiprilat** |
| 1 | 0.26 | 2.92 | 3.18 |
| 2 | 0.16 | 0.80 | 0.96 |
| 3 | 0.26 | 8.23 | 8.49 |
| 4 | 0.08 | 18.65 | 18.73 |
| 5 | 0.06 | 15.45 | 15.51 |
| 6 | 0.13 | 11.81 | 11.94 |
| 7 | 0.08 | 14.80 | 14.88 |
| Tritace® | 0.06 | 1.36 | 1.42 |

## Claims

1. A stable pharmaceutical composition comprising: an intimate admixture including a 2-aza-bicyclo[3.3.0]-octane-3-carboxylic acid derivative and an effective amount of a lubricant to stabilize the composition; and an external excipient.

2. The composition according to claim 1, wherein the intimate admixture is in granular form.

3. The composition according to claim 1 or claim 2, wherein the effective amount of the lubricant ranges from about 0.3 % to about 60% by weight of the intimate admixture, preferably from about 0.8 % to about 50 % by weight of the intimate admixture, more preferably from about 1 % to about 40% by weight of the intimate admixture, most preferably from about 2 % to about 10 % by weight of the intimate admixture.

4. The composition according to any preceding claim, wherein the lubricant is selected from the group consisting of magnesium stearate, talc, stearic acid, glycerylbehenate, polyethylene glycol, ethylene oxide polymers, sodium lauryl sulfate, magnesium lauryl sulfate, sodium oleate, DL-leucine, and sodium stearyl fumarate.

5. The composition according to claim 4, wherein the lubricant is sodium stearyl fumarate.

6. The composition according to any preceding claim, wherein the intimate admixture further comprises one non-lubricant excipient.

7. The composition according to claim 6, wherein the non-lubricant excipient is microcrystalline cellulose.

8. The composition according to claim 7, wherein the non-lubricant excipient is in the amount of about 95% or less by weight of the intimate admixture.

9. The composition according to any preceding claim, further comprising a diuretic agent.

10. The composition according to claim 9, wherein the diuretic agent is hydrochlorothiazide.

11. The composition according to any preceding claim, wherein the derivative is selected from the group consisting of ramipril, quinapril, moexipril, fosinopril, enalapril, perindopril, and trandolapril.

12. The composition according to claim 11, wherein the derivative is ramipril.

13. The composition according to any preceding claim, wherein the derivative is present in an amount of from about 0.3% to about 6% by weight of the total composition, preferably from about 0.8% to about 5% by weight of the total composition, more preferably from about 0.8% to about 4.2% by weight of the total composition.

14. The composition according to any preceding claim, wherein the composition is in solid unit dosage form.

15. The composition according to claim 14, wherein the composition is in tablet form.

16. The composition according to claim 14, wherein the composition is in capsule form.

17. The composition according to any preceding claim, wherein the amount of a principal degradant present in the composition after 48 hours at 55°C is less than 3% by weight of the derivative, preferably the amount of a principal degradant present in the composition after 48 hours at 55°C is less than 1% by weight of the derivative.

18. The composition according to any of claims 1 to 16, wherein the amount of an active form degradant present in the composition after 48 hours at 55°C is less than 0.3% by weight of the derivative, preferably the amount of an active form degradant present in the composition after 48 hours at 55°C is less than 0.2% by weight of the derivative.

19. The composition according to any of claims 1 to 16, wherein the total amount of an active form degradant and a principal degradant present in the composition after 48 hours at 55°C is less than 3.3% by weight of the derivative, preferably the total amount of an active form degradant and a principal degradant present in the composition after 48 hours at 55°C is less than 1% by weight of the derivative.

20. The composition according to any of claims 17 to 19, wherein the active form degradant is ramiprilat, the principal degradant is diketopiperazine, and the derivative is ramipril.

21. A method for preparing a stable pharmaceutical composition comprising: forming an intimate admixture including a 2-aza-bicyclo[3.3.0]-octane-3-carboxylic acid derivative and a lubricant; and blending the intimate admixture with an external excipient.

22. The method of claim 21, further comprising transforming the final blend into solid unit dosage form.

23. The method of claim 22, wherein the composition is in tablet form.

24. The method of claim 22, wherein the composition is in capsule form.

25. The method of claim 21 or 22, wherein the intimate admixture is in granular form.

26. The method of claim 25, wherein the intimate admixture is formed by dry granulation or wet granulation.

27. A 2-aza-bicyclo[3.3.0]-octane-3-carboxylic acid derivative composition made by the process of any of claims 21 to 26.

## Patentansprüche

1. Stabile pharmazeutische Zusammensetzung, welche ein inniges Gemisch, das ein 2-Aza-bicyclo[3.3.0]-octan-3-carbonsäure-Derivat und eine wirksame Menge eines Schmiermittels zum Stabilisieren der Zusammensetzung beinhaltet, sowie einen äußeren Hilfsstoff umfaßt.

2. Zusammensetzung nach Anspruch 1, wobei das innige Gemisch in granulärer Form vorliegt.

3. Zusammensetzung nach Anspruch 1 oder Anspruch 2, wobei die wirksame Menge des Schmiermittels im Bereich von etwa 0,3% bis etwa 60%, bezogen auf das Gewicht des innigen Gemischs, vorzugsweise von etwa 0,8% bis etwa 50%, bezogen auf das Gewicht des innigen Gemischs, bevorzugter von etwa 1% bis etwa 40%, bezogen auf das Gewicht des innigen Gemischs, und am meisten bevorzugt von etwa 2% bis etwa 10%, bezogen auf das Gewicht des innigen Gemischs, liegt.

4. Zusammensetzung nach einem der vorangegangenen Ansprüche, wobei das Schmiermittel aus der Gruppe ausgewählt ist, bestehend aus Magnesiumstearat, Talk, Stearinsäure, Glycerylbehenat, Polyethylenglycol, Ethylenoxidpolymeren, Natriumlaurylsulfat, Magnesiumlaurylsulfat, Natriumoleat, DL-Leucin und Natriumstearylfumarat.

5. Zusammensetzung nach Anspruch 4, wobei das Schmiermittel Natriumstearylfumarat ist.

6. Zusammensetzung nach einem der vorangegangenen Ansprüche, wobei das innige Gemisch weiterhin einen Nicht-Schmiermittel-Wilfsstoff umfaßt.

7. Zusammensetzung nach Anspruch 6, wobei der Nicht-Schmiermittel-Hilfsstoff mikrokristalline Zellulose ist.

8. Zusammensetzung nach Anspruch 7, wobei der Nicht-Schmiermittel-Hilfsstoff in der Menge von etwa 95% oder weniger, bezogen auf das Gewicht des innigen Gemischs, vorliegt.

9. Zusammensetzung nach einem der vorangegangenen Ansprüche, welche weiterhin ein Diuretikum umfaßt.

10. Zusammensetzung nach Anspruch 9, wobei das Diuretikum Hydrochlorthiazid ist.

11. Zusammensetzung nach einem der vorangegangenen Ansprüche, wobei das Derivat aus der Gruppe ausgewählt ist, bestehend aus Ramipril, Chinapril, Moexipril, Fosinopril, Enalapril, Perindopril und Trandolapril.

12. Zusammensetzung nach Anspruch 11, wobei das Derivat Ramipril ist.

13. Zusammensetzung nach einem der vorangegangenen Ansprüche, wobei das Derivat in einer Menge von etwa 0,3% bis etwa 6%, bezogen auf das Gewicht der Gesamtzusammensetzung, bevorzugt von etwa 0,8% bis etwa 5%, bezogen auf das Gewicht der Gesamtzusammensetzung und bevorzugter von etwa 0,8% bis etwa 4,2%, bezogen auf das Gewicht der Gesamtzusammensetzung, vorliegt.

14. Zusammensetzung nach einem der vorangegangenen Ansprüche, wobei die Zusammensetzung in Form einer festen Dosierungseinheit vorliegt.

15. Zusammensetzung nach Anspruch 14, wobei die Zusammensetzung in Tablettenform vorliegt.

16. Zusammensetzung nach Anspruch 14, wobei die Zusammensetzung in Kapselform vorliegt.

17. Zusammensetzung nach einem der vorangegangenen Ansprüche, wobei die Menge eines in der Zusammensetzung vorliegenden Hauptabbaustoffs nach 48 Stunden bei 55°C weniger als 3 Gewichts-% des Derivats ausmacht und bevorzugt die Menge eines in der Zusammensetzung vorliegenden Hauptabbaustoffs nach 48 Stunden bei 55°C weniger als 1 Gewichts-% des Derivats ausmacht.

18. Zusammensetzung nach einem der Ansprüche 1 bis 16, wobei die Menge einer in der Zusammensetzung vorliegenden aktiven Form eines Abbaustoffs nach 48 Stunden bei 55°C weniger als 0,3 Gewichts-% des Derivats ausmacht und bevorzugt die Menge einer in der Zusammensetzung vorliegenden aktiven Form eines Abbaustoffs nach 48 Stunden bei 55°C weniger als 0,2 Gewichts-% des Derivats ausmacht.

19. Zusammensetzung nach einem der Ansprüche 1 bis 16, wobei die Gesamtmenge einer aktiven Form eines Abbaustoffs und eines Hauptabbaustoffs, die in der Zusammensetzung vorliegen, nach 48 Stunden bei 55°C weniger als 3,3 Gewichts-% des Derivats ausmacht und bevorzugt die Gesamtmenge einer aktiven Form eines Abbaustoffs und eines Hauptabbaustoffs, die in der Zusammensetzung vorliegen, nach 48 Stunden bei 55°C weniger als 1 Gewichts-% des Derivats ausmacht.

20. Zusammensetzung nach einem der Ansprüche 17 bis 19, wobei die aktive Form des Abbaustoffs Ramiprilat ist, der Hauptabbaustoff Diketopiperazin ist und das Derivat Ramipril ist.

21. Verfahren zur Herstellung einer stabilen pharmazeutischen Zusammensetzung, welches das Bilden eines innigen Gemischs, das ein 2-Aza-bicyclo[3.3.0]-octan-3-carbonsäure-Derivat und ein Schmiermittel beinhaltet, und das Vermischen des innigen Gemischs mit einem äußeren Hilfsstoff umfaßt.

22. Verfahren nach Anspruch 21, welches weiterhin das Umwandeln des Endgemischs in die Form einer festen Dosierungseinheit umfaßt.

23. Verfahren nach Anspruch 22, wobei die Zusammensetzung in Tablettenform vorliegt.

24. Verfahren nach Anspruch 22, wobei die Zusammensetzung in Kapselform vorliegt.

25. Verfahren nach Anspruch 21 oder 22, wobei das innige Gemisch in granulärer Form vorliegt.

26. Verfahren nach Anspruch 25, wobei das innige Gemisch durch Trockengranulation oder Feuchtgranulation gebildet wird.

27. 2-Aza-bicyclo[3.3.0]-octan-3-carbonsäure-Derivat-Zusammensetzung, hergestellt durch das Verfahren nach einem der Ansprüche 21 bis 26.

## Revendications

1. Une composition pharmaceutique stable comprenant; un mélange intime incluant un dérivé d'acide 2-aza-bicyclo[3.3.0]-octane-3-carboxylique et une quantité efficace d'un lubrifiant pour stabiliser la composition; et un excipient externe.

2. La composition selon la revendication 1, dans laquelle le mélange intime est sous forme granulaire.

3. La composition selon la revendication 1 ou la revendication 2, dans laquelle la quantité efficace du lubrifiant est comprise entre environ 0,3% et environ 60% en poids du mélange intime, de préférence entre environ 0,8% et environ 50% en poids du mélange intime, encore plus préférentiellement entre environ 1% et environ 40% en poids du mélange intime, plus particulièrement encore entre environ 2% et environ 10% en poids du mélange intime

4. La composition selon une quelconque des revendications précédentes, dans laquelle le lubrifiant est choisi dans le groupe comprenant le stéarate de magnésium, le talc, l'acide stéarique, le glycerylbéhénate, le polyéthylène glycol, les polymères d'oxyde d'éthylène, le laurylsulfate de sodium, le lauryl sulfate de magnésium, l'oléate de sodium, la DL-Ieucine et le fumarate de stéaryl sodium.

5. La composition selon la revendication 4, dans laquelle le lubrifiant est le fumarate de stéaryl sodium.

6. La composition selon une quelconque des revendications précédentes, dans laquelle le mélange intime comprend en outre un excipient non lubrifiant

7. La composition selon la revendication 6, dans laquelle l'excipient non lubrifiant est la cellulose microcristalline.

8. La composition selon la revendication 7, dans laquelle l'excipient non lubrifiant est employé en une quantité d'au plus 95% en poids par rapport au mélange intime.

9. La composition selon une quelconque des revendications précédentes, comprenant en outre un agent diurétique.

10. La composition selon la revendication 9, dans laquelle l'agent diurétique est l'hydrochlorothiazide.

11. La composition selon une quelconque des revendications précédentes, dans laquelle le dérivé est choisi dans le groupe comprenant ramipril, quinapril, moexipril, fosinopril, enalapril, perindopril, et trandolapril.

12. La composition selon la revendication II, dans laquelle le dérivé est le ramipril.

13. La composition selon une quelconque des revendications précédentes, dans laquelle le dérivé est présent en une quantité comprise entre environ 0,3% et environ 6% en poids de la composition totale, de préférence entre environ 0,8% et environ 5% en poids de la composition totale, plus particulièrement de préférence entre environ 0,8% et environ 4,2% en poids de la composition totale.

14. La composition selon une quelconque des revendications précédentes, dans laquelle la composition est sous une forme de dose pharmaceutique unitaire solide.

15. La composition selon la revendication 14, dans laquelle la composition est sous forme de comprimés.

16. La composition selon la revendication 14, dans laquelle la composition est sous forme de gélule.

17. La composition selon une quelconque des revendications précédentes, dans laquelle la quantité de l'agent dégradant principal présent dans la composition après 48 heures à 55°C est inférieure à 3% en poids du dérivé, la quantité de l'agent dégradant principal présent dans la composition après 48 heures à 55°C étant de préférence inférieure à 1% en poids du dérivé.

18. La composition selon une quelconque des revendications 1 à 16, dans laquelle la quantité de l'agent dégradant principal présent dans la composition après 48 heures à 55°C est inférieure à 0,3% en poids du dérivé, la quantité de l'agent dégradant principal présent dans la composition après 48 heures à 55°C étant de préférence inférieure à 0,2% en poids du dérivé.

19. La composition selon une quelconque des revendications 1 à 16, dans laquelle la quantité totale de la forme active du dégradant et du dégradant principal présent dans la composition après 48 heures à 55°C est inférieure à 3,3% en poids du dérivé, la quantité totale de la forme active du dégradant et du dégradant principal présent dans la composition après 48 heures à 55°C étant de préférence inférieure à 1% en poids du dérivé.

20. La composition selon une quelconque des revendications 17 à 19, dans laquelle la forme active du dégradant est le ramiprilat, le dégradant principal est la dicétopipérazine et le dérivé est le ramipril.

21. Un procédé pour préparer une composition pharmaceutique stable comprenant la formation d'un mélange intime incluant un dérivé d'acide 2-aza-bicyclo[3.3.0]-octane-3-carboxylique et d'un lubrifiant; et comprenant l'homogénéisation du mélange intime avec un excipient externe.

22. Le procédé selon la revendication 21, comprenant en outré la transformation de l'homogénéisât final en une forme pharmaceutique unitaire solide.

23. Le procédé selon la revendication 22, dans laquelle la composition sous forme de comprimés.

24. Le procédé selon la revendication 22, dans laquelle la composition est sous forme de gélule.

25. Le procédé selon la revendication 21 ou 22, dans laquelle le mélange intime est sous forme granulaire.

26. Le procédé selon la revendication 25, dans laquelle le mélange intime est obtenu par dry granulation à l'état sec ou par granulation à l'état humide.

27. Une composition de dérivé d'acide 2-aza-bicyclo[3.3.0]-octane-3-carboxylique obtenue par le procédé selon une quelconque des revendications 21 à 26.
